# FASCICULE DE BREVET EUROPEEN

(11) **EP 3 661 482 B1**
(45) Date de publication et mention de la délivrance du brevet: **22.09.2021**
(21) Numéro de dépôt: 18746960.6
(22) Date de dépôt: 03.08.2018
(51) Int. Cl.: A61K 8/49, A61Q 11/00, A61K 8/69, A61K 8/97, A61K 8/21, A61K 31/455, A61K 36/61

(54) **COMPOSITION COMPRENANT UN EXTRAIT DE MYRTE ET UN SEL DE FLUOR DESTINEE A UNE APPLICATION BUCCO-DENTAIRE**
EINE MYRTEEXTRAKT UND FLUORIDSALZ ENTHALTENDE ZUSAMMENSETZUNG ZUR MUNDPFLEGE
COMPOSITION COMPRISING A MYRTLE EXTRACT AND A FLUORIDE SALT FOR ORAL CARE APPLICATION

(30) Priorité: 04.08.2017 FR 1757537
(43) Date de publication de la demande: 10.06.2020
(73) Titulaire: Pierre Fabre Médicament, 92100 Boulogne-Billancourt (FR)
(72) Inventeur: FIORINI, Christel, 31500 Toulouse (FR); FABRE, Bernard, 31450 Belberaud (FR)
(74) Mandataire: Regimbeau
(86) Numéro de dépôt international: PCT/EP2018/071204
(87) Numéro de publication internationale: WO 2019/025622

(56) Documents cités:
- WO-A1-2008/065382
- FR-A1- 2 318 632
- FR-A1- 2 915 386
- DATABASE GNPD [Online] MINTEL; décembre 2016 (2016-12), "Toothpaste with Organic Mint", XP002775755, Database accession no. 4517187
- "MAZMAZAH-E- AAS", TKDL,, 1 janvier 1977 (1977-01-01), XP003036097,
- "MAZMAZAH-E- AAS WARAN BARA-E- LESSA", TKDL,, 1 janvier 1977 (1977-01-01), XP003036096,

## Description

La présente invention concerne une composition buccale comprenant un extrait de myrte et un sel de fluor ainsi que son utilisation pour le traitement d'affections bucco-dentaires notamment pour prévenir et/ou diminuer la formation et l'accumulation du biofilm dentaire.

La cavité buccale constitue une véritable niche écologique par laquelle transitent et séjournent la salive, les aliments, l'air et les produits bactériens.

Dans la cavité buccale, les bactéries adoptent deux modes de vie différents : soit elles survivent à l'état planctonique, dans lequel les organismes isolés flottent dans le milieu buccal, soit elles forment un biofilm en s'attachant à la surface dentaire et en vivant en se développant en communauté.

La santé dentaire et parodontale repose sur un état d'équilibre dans lequel la population bactérienne coexiste avec l'hôte sans aucun dommage pour les tissus de ce dernier. La rupture de cet équilibre, c'est-à-dire quand la composition et/ou les activités métaboliques des communautés du biofilm dentaire sont perturbées, peut entrainer de nombreuses affections bucco-dentaires en particulier les lésions du parodonte.

Les lésions du parodonte apparaissent généralement d'abord au niveau de la gencive, provoquant des gingivites, inflammations de la gencive marginale. Puis, elles peuvent évoluer en parodontites affectant l'ensemble du parodonte et deviennent irréversibles en l'absence de traitement.

Il existe différentes formes de gingivites : la gingivite associée à la présence de plaque dite banale de négligence, la gingivite aiguë ulcéro-nécrotique et la gingivite dont l'origine n'est pas liée à la plaque (associée à une maladie cutanée ou limitée à la gencive et/ou à l'ensemble de la muqueuse buccale, allergique ou infectieuse).

Le biofilm dentaire (ou plaque dentaire) est une accumulation hétérogène, adhérente à la surface des dents ou située dans l'espace gingivo-dentaire, composée d'une communauté microbienne riche en bactéries aérobies ou anaérobies, enrobée d'une matrice intercellulaire de polymères d'origine microbienne et salivaire. Il s'agit d'une organisation bactérienne complexe dont les premiers stades de formation correspondent à un dépôt de glycoprotéines sur les surfaces des tissus durs ou des tissus mous baignant dans la salive. Cette première couche porte le nom de pellicule exogène acquise (PEA) ou encore « biofilm salivaire ». Elle est secondairement colonisée par des micro -organismes qui vont s'organiser en fonction de critères physicochimiques, nutritionnels ou relationnels et constituer ainsi un dépôt mou, adhérent, tenace, et de couleur blanc jaunâtre, à la surface des dents et des matériaux dentaires couramment utilisés.

On distingue deux types de plaque définis en fonction de leur localisation anatomique par rapport à la gencive : la plaque supragingivale et la plaque sous-gingivale. Ces deux types de plaques constituent deux microenvironnements différents. L'environnement supragingival est baigné par la salive, tandis que l'environnement sous-gingival est baigné par le fluide du sillon gingival. L'environnement supragingival est surtout aérobie, alors que l'environnement sous-gingival est presque exclusivement anaérobie. L'espace sous-gingival a la forme d'un cul-de-sac, sans chasse liquidienne, et les forces mécaniques susceptibles de désagréger les populations bactériennes établies y sont rares. Au contraire, les zones supragingivales sont balayées continuellement par la salive, exposées à tous les mécanismes d'attrition propres à la cavité buccale (mastication, déglutition, phonation) et directement accessibles aux mesures d'hygiène.

La plaque supragingivale et la plaque sous-gingivale se distinguent également par leur potentiel pathogénique : la plaque supragingivale est spécifiquement impliquée dans la pathologie carieuse alors que la plaque sous-gingivale est associée aux pathologies gingivales et parodontales. Les principales bactéries retrouvées dans la plaque supragingivale sont *Streptococcus mutans, Streptococcus sanguis, Streptococcus mitis, Streptococcus sobrinus, Lactobacillus sp., et Actinomyces sp. (A. viscosus).* En fonction de sa situation côté dent ou côté épithélium, la composition de la flore sous-gingivale varie de façon importante. On note la présence de *Porphorymonas gingivalis* et de *Fusobacterium nucleatum,* qui sont fortement impliquées dans les pathologies parodontales.

Des agents antiseptiques bactéricides sont souvent utilisés dans des compositions hygiéniques pour réduire la plaque dentaire. Toutefois, les agents bactéricides ne sont pas complètement satisfaisants car leur action antiseptique forte peut induire un déséquilibre au sein de la population bactérienne naturelle de la cavité buccale. En effet, les produits antiseptiques agissent sur les microorganismes de manière drastique et rapide. Ils risquent par ailleurs d'induire des résistances lorsqu'ils sont utilisés trop fréquemment.

La société Lavera commercialise un dentifrice contenant du fluor et un hydrolat de myrte. Un hydrolat correspond à l'eau distillée végétale obtenue par entraînement à la vapeur d'eau de diverses parties de plantes aromatiques ou non. Il représente la phase aqueuse polaire, recondensée et séparée de l'huile essentielle.

Le document FR2915386 décrit un extrait aqueux acide de pépins de raisins riche en polyphénols, c'est-à-dire contenant au moins 50% voire 90% de polyphénols, en association avec un sel de fluor dans le traitement des maladies bucco-dentaires.

Le document FR2318632 décrit un sel de fluor et son action inhibitrice sur la plaque dentaire.

Le document WO2008/065382 décrit l'utilisation de myrte pour la traitement de la plaque dentaire chez les animaux de compagnie. Ce document évoque la myrte et différents types d'extrait sans préciser ni leur qualité ni le mode d'obtention et encore moins l'activité qui y est associée.

Il existe donc un besoin de développer des agents capables de limiter l'adhésion bactérienne (et par voie de conséquence le développement du biofilm), permettant ainsi de conserver une bonne santé bucco-dentaire sans effets secondaires et sans perturber l'équilibre de l'écosystème buccal. Ces agents pourront agir au niveau des interactions de la cellule bactérienne avec l'ensemble des surfaces buccales : surfaces dures (ex. émail et/ou dentine), muqueuses, ainsi qu'entre cellules bactériennes.

Il a été découvert de manière surprenante que l'association d'un extrait de myrte avec un sel de fluor permet d'obtenir une action synergique efficace anti-adhésion assurant in fine une action anti-biofilm respectueux de l'équilibre de la flore bactérienne buccale.

La présente invention a donc pour objet une composition comprenant un extrait de myrte et au moins un sel de fluor.

En particulier, une telle composition est destinée à une application buccale.

Le sel de fluor selon la présente invention est notamment choisi parmi les fluorures de potassium, de sodium et d'étain, les fluorures d'amines et les fluorures d'ammonium quaternaire.

De manière préférée, le sel de fluor selon l'invention est un fluorure d'amine de formule (I) suivante : 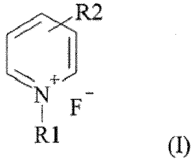 dans laquelle :
R1 représente un atome d'hydrogène ou un groupe (C₁-C₆)alkyle ;
R2 représente un groupe CH₂OH, CO₂((C₁-C₆)alkyle), CONH(CH₂)₂OH ou 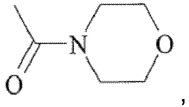
et R2 se trouve en position 2 ou 3 par rapport à l'atome d'azote.

Par groupement « (C₁-C₆)alkyle », on entend, au sens de la présente invention, une chaîne hydrocarbonée saturée, linéaire ou ramifiée, comportant 1 à 6, notamment 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Dans un mode de réalisation, R1 est H.

Dans un mode de réalisation particulièrement préféré, le sel de fluor selon l'invention est le fluorhydrate de nicométhanol (fluorinol®), qui correspond à un composé de formule (I) dans laquelle : R1=H et R2=CH₂OH est en position 3 par rapport à l'azote.

L'extrait de myrte selon l'invention peut être obtenu à partir d'une plante de la famille des Myrtaceae, plus particulièrement du genre Myrtus et plus précisément de l'espèce *Myrtus communis,* dont l'espèce *Myrtus communis.*

L'extrait de myrte selon l'invention peut être extrait de la plante entière ou d'une ou plusieurs parties de la plante notamment des parties aériennes, des feuilles et/ou des fleurs et/ou des fruits par toutes les méthodes connues de l'homme du métier. Les feuilles, les parties aériennes et les fruits utilisés seront préférentiellement secs.

Lorsqu'un mélange de fruit et de feuilles est utilisé, le rapport en poids feuilles / fruits pourra être compris entre 60/40 et 95/5, par exemple être égal à 90/10.

Dans un mode de réalisation l'extrait est obtenu à partir des feuilles uniquement.

Cet extrait pourra notamment être obtenu par extraction avec des solvants.

La fabrication d'un extrait de myrte selon l'invention peut comprendre les étapes suivantes :
- broyage de la plante ou de parties de la plante, telles que les feuilles, les parties aériennes, les fleurs et/ou les fruits de myrte ;
- extraction des parties aériennes, des feuilles et/ou des fruits broyés par un solvant, notamment par un solvant polaire ;
- récupération de la solution d'extraction par séparation solide/liquide, notamment par filtration, centrifugation ou essorage ;
- concentration de la solution récupérée par évaporation ou séchage de l'extrait (par évaporation poussée ou lyophilisation) ; et
- standardisation en extrait sec par ajout de support.

Le solvant polaire selon l'invention pourra notamment être de l'eau, un alcool en C₁-C₄, l'acétone, un mélange eau-alcool, un mélange acétone-eau ou un mélange eau-propylène glycol. Dans un mode de réalisation, le solvant polaire est un mélange eau/éthanol, notamment un mélange eau/éthanol à 50 % (v/v).

Dans un mode de réalisation, le rapport en poids de la plante ou des parties de la plante sera compris entre 1 et 20, notamment entre 1 et 4 en poids par rapport au poids du solvant.

La température d'extraction pourra être comprise entre une température ambiante (entre 10 °C et 35 °C) et la température d'ébullition du solvant ou la température de l'eau à l'état subcritique (120 °C à 220 °C).

La durée d'extraction, notamment d'une extraction à reflux est typiquement comprise entre 1h et 24h. La concentration de la solution récupérée par évaporation pourra être réalisée à pression réduite. La température d'évaporation dépendra du solvant utilisé et pourra être comprise entre 40 et 100 °C.

Dans un mode de réalisation, l'évaporation est prolongée jusqu'à l'obtention d'une poudre. La poudre peut alors être solubilisée pour obtenir un extrait liquide.

Dans un autre mode de réalisation, on peut, lors de l'étape d'évaporation, ajouter un solvant à haut point d'ébullition tel que la glycérine, le propylène glycol, le butylène glycol ou le transcutol, notamment le propylène glycol. Après évaporation de l'eau et du solvant, l'extrait obtenu est alors sous forme d'extrait liquide.

L'extrait liquide selon l'invention comprendra entre 1 % et 50 % d'extrait sec de myrte (p/p), notamment entre 8 % et 12 %, notamment 10 %. L'extrait de myrte selon l'invention pourra comprendre notamment les composés suivants :
- Une huile essentielle composée notamment d'acétate de myrtényle, de 1,8 cinéole, de linalol, d' *α*-pinène, de limonène, de myrto et de myrtenol ;
- Des composés phénoliques : notamment :
   - des tanins, en particulier des tanins condensés et/ou des ellagitannins (dont l'oenothéine B, l'eugeniflorine D2, les tellimagrandines I et II, et les galloyl-glucosides),
   - des flavonoïdes, notamment un ou plusieurs composés choisis parmi le kaempférol, la quercétine et ses dérivés glycosylés (quercétine 3-galactoside, quercétine 3-glucoside, quercétine 6"-O-galloyl galactoside), la myricétine et ses dérivés glycosylés (myricétine 3-rhamnoglucoside, myricétine 3'-glucoside, myricétine 3-rhamnoside, myricétine 3-galactoside, myricétine 3,3'- digalactoside, myricétine 3-O-β-d-xyloside, myricétine 6"-O-galloyl galactoside), la tectochrysine et la sideroxyline,
   - des coumarines (dont l'esculétol et l'esculoside), et/ou
   - des acylphloroglucinols (dont les myrtucommulones) ; et
- Des acides phénoliques,
- Des triterpènes et stérols, notamment un ou plusieurs composés choisis parmi des dérivés de l'acide usnique (usnone A), le bétulinol, cearoine (2,5-dihydroxy-4-methoxybenzophénone), l'érythrodiol, l'acide ursolique, l'acide oléanolique, l'acide corosolique, l'acide arjunolique et le β-sitostérol.

Dans un mode de réalisation, l'extrait de myrte selon l'invention comprendra entre 1 % et 50 %, notamment entre 10 % et 40 % ou entre 20 % et 30 % de composés phénoliques en poids sec par rapport au poids total de la matière sèche.

Parmi les composés phénoliques, on entend notamment citer les flavonoïdes, les acylphloroglucinols et les tanins.

Dans un mode de réalisation, l'extrait de myrte selon l'invention comprendra entre 0,1 et 10 %, notamment entre 1 et 5 % ou entre 2 et 3 % de flavonoïdes en poids sec par rapport au poids total de la matière sèche.

La quantité de flavonoïdes pourra être mesurée par toutes les méthodes bien connues de l'homme du métier et notamment celles décrites dans la Pharmacopée européenne (01/2015:2427).

Dans un mode de réalisation, l'extrait de myrte selon l'invention comprendra entre 0,15 et 30%, notamment entre 0,5 et 10%, notamment entre 1 et 5 % de tanins en poids sec par rapport au poids total de la matière sèche.

La quantité de tanins pourra être mesurée par toutes les méthodes bien connues de l'homme du métier et notamment celles décrites dans la Pharmacopée européenne (01/2008:20814).

Dans un mode de réalisation, l'extrait de myrte selon l'invention comprendra entre 0,1 et 30 %, notamment entre 0,5 et 10 %, notamment entre 1 et 5 % de triterpènes en poids sec par rapport au poids total de la matière sèche.

La quantité de triterpènes pourra être mesurée par toutes les méthodes bien connues de l'homme du métier et notamment celles décrites dans la Pharmacopée européenne.

Dans un mode de réalisation, l'extrait de myrte selon l'invention comprendra entre 1 et 5 %, notamment entre 2 et 3 % de flavonoïdes en poids sec par rapport au poids total de la matière sèche dudit extrait et/ou entre 0,5 et 10 %, notamment entre 1 et 5 % de tanins en poids sec par rapport au poids total de la matière sèche dudit extrait et/ou entre 0,5 et 5 % notamment entre 1 et 5 % de triterpènes en poids sec par rapport au poids total de la matière sèche dudit extrait.

De manière préférée, l'extrait selon l'invention comprend entre 1 et 5 %, de flavonoïdes, entre 0,5 et 10 % de tanins, entre 0,5 et 5 % de triterpènes en poids sec par rapport au poids total de la matière sèche dudit extrait.

Par flavonoïdes selon l'invention on entend notamment les flavonoïdes naturellement présents dans la myrte et notamment le kaempférol, le myricétol et ses glycosides, la quercétine et ses glycosides, la tectochrysine et la sideroxyline.

Par tanins selon l'invention on entend les tanins naturellement présents dans la myrte et notamment les tanins condensés et hydrolysables (comme l'oenothéine B, l'eugeniflorine D2 et les tellimagrandines I et II), les ellagitannins et composés apparentés tels que l'acide gallique et l'acide 3,5-di-O-gallate.

Par acylphloroglucinols on entend notamment les myrtucommulones A, B', B, C, D, E F, S, isoS.

Selon un mode de réalisation, la composition selon l'invention comprend des quantités synergiques d'extrait de myrte et de sel de fluor. Par synergique, on entend un effet supérieur de la combinaison d'extrait de myrte et de sel de fluor en comparaison aux effets additionnés de deux compositions identiques mais ne comprenant respectivement que l'extrait de myrte ou que le sel de fluor. La synergie pourra notamment être évaluée par mesure de l'effet de la composition sur l'adhésion bactérienne à la surface des dents (ou d'une surface mimant les dents) notamment comme décrit dans les exemples de la présente demande. L'effet synergique sera notamment constaté lorsque la composition selon l'invention a un effet anti-adhésion supérieur à un log, notamment supérieur à 2 log, notamment compris entre 1 et 3 log, par rapport aux effets anti-adhésion additionnés de deux compositions identiques mais ne comprenant respectivement que le sel de fluor et que l'extrait de myrte.

Dans un mode de réalisation, la composition selon l'invention comprend une quantité d'extrait de myrte comprise entre 0,005 % et 3 % d'extrait sec de myrte (p/p), notamment entre 0,01 % et 1 %, notamment de 0,02 % en poids par rapport au poids total de la composition.

Dans un mode de réalisation, la composition selon l'invention comprend une quantité de sel de fluor, notamment le fluorhydrate de nicométhanol, comprise entre 1 % et 10 % (p/p), notamment entre 3 % et 7 %, notamment 5 % en poids par rapport au poids total de la composition.

Dans un mode de réalisation, la composition selon l'invention comprend entre 50 ppm et 10 000 ppm de fluor, notamment entre 1 000 ppm et 2 000 ppm, notamment 1 350 ppm.

Dans un mode de réalisation, la composition selon l'invention comprend une quantité d'extrait de myrte comprise entre 0,005 et 2 % d'extrait sec de myrte (p/p), notamment entre 0,01 et 1 %, notamment de 0,02 % et une quantité de sel de fluor, notamment le fluorhydrate de nicométhanol, comprise entre 1 et 10 % (p/p), notamment entre 3 et 7 %, notamment 5 %.

Dans un mode de réalisation, la composition selon l'invention comprend une quantité d'extrait de myrte comprise entre 0,005 et 2 % d'extrait sec de myrte (p/p), notamment entre 0,01 et 1 %, notamment de 0,02 % et entre 50 et 10 000 ppm de fluor, notamment entre 1000 et 2 000 ppm, notamment 1 350 ppm.

Dans un mode de réalisation, la composition selon l'invention comprend entre 0,005 et 1 % de triterpènes et/ou entre 0,005 et 1 % de flavonoïdes et/ou entre 0,005 et 1 % de tanins. Par exemple entre 0,01 et 0,05 de triterpènes et/ou entre 0,01 et 0,05 % de flavonoïdes et/ou entre 0,01 et 0,05 % de tanins (en p/p par rapport à la composition). De manière préférée la composition selon l'invention comprend entre 0,005 et 1 % de triterpènes, entre 0,005 et 1 % de flavonoïdes et entre 0,005 et 1 % de tanins (en p/p par rapport à la composition). Plus particulièrement encore entre 0,01 et 0,05 de triterpènes, entre 0,01 et 0,05 % de flavonoïdes et entre 0,01 et 0,05 % de tanins (en p/p par rapport à la composition).

Par composition buccale ou composition destinée à une application buccale selon l'invention, on entend toute composition formulée de manière à pouvoir être administrée dans la cavité buccale.

La composition selon la présente invention peut notamment se présenter sous la forme d'un bain de bouche, d'un gel ou d'une pâte dentifrice, d'un gel gingival, d'une gomme à mâcher, d'une pastille à sucer, d'un fil dentaire, d'une capsule extrudée, de brins pour brosses à dents, d'un spray, d'un pansement adhésif, d'une lingette imprégnée, d'une pâte adhésive pour dentiers et prothèses ou d'une pâte prophylactique de polissage.

Dans un mode de réalisation, la composition buccale selon l'invention est sous forme d'une pâte dentifrice, d'un gel, d'un spray ou d'un bain de bouche, avantageusement sous la forme d'une pâte dentifrice ou d'un bain de bouche.

Dans un mode de réalisation préféré, la composition selon l'invention est sous forme de dentifrice (autrement appelé pâte dentifrice). Elle peut alors comprendre tout excipient pharmaceutiquement acceptable bien connu de l'homme du métier comme apte à entrer dans ce type de composition.

La composition sous forme de dentifrice selon l'invention pourra notamment comprendre : un agent de type moussant tel qu'un sulfate, en particulier le laurylsulfate de sodium, des enzymes, des vitamines, des minéraux tel le calcium, des arômes, des agents polissants tels que des silices, du bicarbonate de sodium ou des phosphates de calcium, des conservateurs, des colorants et/ou des agents épaississants.

La composition sous forme de dentifrice selon l'invention pourra en outre comprendre d'autres principes actifs comme des agents antibactériens, tels que le triclosan ou la chlorexidine, ou des agents anti-biofilm tel qu'un extrait de canneberge (ou Vaccinium niacrocarpon).

Dans un mode de réalisation, la composition selon l'invention ne comprend pas d'autres principes actifs que le sel de fluor et l'extrait de myrte.

La présente invention concerne, en outre, l'utilisation d'un extrait de myrte et d'au moins un sel de fluor pour la préparation d'une composition, notamment une composition buccale.

La présente invention porte, en outre, sur une composition selon l'invention pour son utilisation en tant que médicament.

La présente invention porte en outre sur l'utilisation d'une composition selon l'invention pour la fabrication d'un médicament.

La présente invention porte, en outre, sur une méthode de traitement dans laquelle est administrée une composition selon l'invention.

Dans un mode de réalisation, la composition selon l'invention est utilisée pour prévenir la formation de la plaque dentaire et/ou réduire la plaque dentaire.

La prévention de la formation de la plaque dentaire et/ou la réduction de la plaque dentaire peuvent être évaluées par toute méthode connue de l'homme du métier. Elles peuvent notamment être estimées par mesure de l'adhésion bactérienne à la surface des dents (ou d'un matériau mimant les dents tels que l'hydroxyapatite) en présence et en absence de la composition selon l'invention. La réduction de la plaque dentaire peut alternativement être mise en évidence par l'utilisation d'agents révélateurs de plaque (Disclosing agent).

Par mesure de l'adhésion bactérienne on peut notamment mesurer celle d'une ou plusieurs espèces bactériennes, dont les espèces dites pionnières dans la succession écologique du biofilm dentaire, comprises ou consistant en la liste suivante : *Streptococcus oralis, Streptococcus gordonii, Streptococcus mutans, et Actinoyces naeslundii.*

L'adhésion bactérienne pourra notamment être mesurée par la méthode décrite dans les exemples de la présente demande.

### EXEMPLES :

### MATERIELS ET METHODES :

### Matériels :

Bactéries : une suspension mixte bactérienne à 10⁷ UFC/mL est obtenue par mélange d'une quantité égale de bactéries des quatre espèces suivantes : *Streptococcus oralis, Streptococcus gordonii, Streptococcus mutans, et Actinoyces naeslundii.*

Entretien des souches et dénombrement bactérien : les souches bactériennes sont cultivées sur milieu gélosé Columbia + 5 % de sang de mouton stérile à 16 °C en atmosphère anaérobie.

Salive : des pools de salives humaines sont constitués et filtrés sur membrane 0,45 µm.

Pastille d'hydroxyapatite (HAP) : des pastilles d'hydroxyapatite (diamètre de 9,4 mm, épaisseur de 1,8 mm) sont stérilisés par autoclavage à 121 °C pendant 15-20 minutes.

Milieu pour la formation du biofilm (BBM) : un bouillon 10X est préparé avec la composition suivante : FeSO₄, 7H₂O (0,005 g/L) ; Na₂HPO₄ (12,5 g/L) ; KH₂PO₄ (5 g/L) ; (NH₄)₂SO₄ (1 g/L) ; glucose (0,5 g/L) et MgSO₄ (100 x 20 g/L). Ce bouillon est reconstitué au moment de l'essai pour obtenir une concentration X (Bouillon BBM).

Produits : L'extrait de myrte est obtenu à partir des feuilles sèches de la plante. Les feuilles sont broyées puis extraites à reflux par un mélange d'eau et d'éthanol à 50 % (v/v). Après filtration, le filtrat est concentré afin d'éliminer l'alcool et l'eau. L'extrait est ensuite solubilisé dans du propylène glycol jusqu'à obtenir une extrait liquide à 10 % d'extrait sec de myrte (p/p). On utilise environ 400 g de feuille pour la fabrication d'un kg d'extrait. L'extrait est un liquide marron-verdâtre renfermant 2 % de triterpènes, 2,5 % de flavonoïdes et 2 % de tanins (p/p, mesurés selon les techniques référencées dans la pharmacopée européenne).

L'extrait de myrte est dilué dans de l'eau distillée à une concentration de 2 %. Le fluorinol à 1 350 ppm est obtenu par dilution de 0,503 g de fluorhydrate de nicométhanol dans 9,5 g d'eau distillée stérile. La solution de fluorinol à 1 350 ppm et d'extrait de myrte est obtenue par mélange dans 9,3 ml d'eau distillée stérile de 0,503 g de fluorhydrate de nicométhanol et de 0,2 mL d'extrait de myrte.

### Méthodes :

Les disques d'HAP stérilisés sont placés dans les puits d'une microplaque stérile 24 puits. Un conditionnement de la surface est réalisé par recouvrement avec 1 mL d'un pool de salive humaine pendant une heure à température ambiante.

Les pastilles sont récupérées (sans rinçage) et sont mises en contact à température ambiante avec les produits seuls ou l'association des deux pendant 5 minutes. Les pastilles ainsi traitées sont déposées dans un puit contenant 1,8 mL de BBM et 200 µL de suspension mixte à 10⁷ UFC/mL pendant 48h à 36 °C en anaérobiose (le milieu BBM est renouvelé à 24h). Afin de simuler des brossages réguliers, les pastilles sont récupérées et mises en contact à température ambiante avec les produits seuls ou l'association des deux pendant 5 minutes aux temps 2h, 6h, 24h, 30h et 48h, puis réintroduites dans le puits comprenant la suspension bactérienne.

Des expériences témoins sont réalisées dans les mêmes conditions dans lesquelles les produits sont remplacés par un volume équivalent d'eau distillée ou de propylène glycol à 1,8 % massique dans de l'eau.

Après 24 ou 48h d'incubation, la détermination du nombre de bactéries viables ayant adhérées à la surface des pastilles d'HAP est réalisée de la façon suivante : les pastilles sont prélevées et rincées avec de l'eau distillée (2 x 2 mL) afin d'éliminer les bactéries non adhérées. La surface des pastilles est grattée dans 2 mL d'eau distillée stérile. Les suspensions obtenues sont homogénéisées (vortex) et des séries de dilution de raison 10 sont réalisées. Les dénombrements sont effectués par étalement de 100 µL de chacune des dilutions sur gélose Columbia + 5 % de sang de mouton stérile.

Après 72 à 96h d'incubation à 36 °C en anaérobiose, les colonnes sont dénombrées sans distinction de l'espèce. Les valeurs obtenues sont transformées en logarithmes décimaux avant d'effectuer l'analyse comparative.

### RESULTATS :

Les expériences réalisées ont pour but d'évaluer in vitro l'activité de l'extrait de myrte et/ou du fluorinol sur la formation de la plaque dentaire.

Pour cela, un modèle classique d'évaluation de la formation de la plaque dentaire est utilisé. Les pastilles d'hydroxyapatites sont utilisées pour mimer la surface dentaire. Les espèces bactériennes utilisées : *Streptococcus oralis, Streptococcus gordonii, Streptococcus mutans, et Actinoyces naeslundii* sont représentatives d'un début de plaque dentaire.

L'adhésion des bactéries à la surface de l'HAP est évaluée en présence ou en absence des produits par mesure du nombre de bactéries adhérentes à 24 et 48h après incubation dans le milieu bactérien (UFC/Pastille) et après des incubations de 5 minutes avec les produits.

Les résultats suivants ont été obtenus :

**Tableau 1 : nombre d'UFC adhérents à la surface de l'HAP après incubations avec les produits pendant 5 minutes.**

| Produits/ UFC/pastilles (Log UFC/pastilles) | 24h | 48h |
|---|---|---|
| Témoin eau distillée | 2,64E+05 (5,42) | 4,58E+04 (4,66) |
| Témoin Propylène glycol 1,8 % | 2,82E+05 (5,45) | 2,88E+04 (4,46) |
| Fluorinol 1 350 ppm | 1,22E+05 (5,09) | 6,00E+04 (4,78) |
| Extrait de myrte 2 % | 1,28E+05 (5,11) | 1,44E+04 (4,16) |
| Fluorinol 1 350 ppm + extrait de myrte 2 % | 5,12E+03 (3,71) | 6,00E+01 (1,78) |

En conclusion les résultats obtenus montrent que :
Le propylène glycol à 1,8 % ne présente pas d'activité sur la formation du biofilm.

Le fluorinol 1 350 ppm ne présente pas d'activité significative sur la formation du biofilm et ce quel que soit le nombre de traitements (3 traitements pour le temps 24h et 6 pour le temps 48h).

L'extrait de myrte à 2 % présente une activité faible sur le biofilm de l'ordre de 0,3-0,5 log. Le temps d'incubation de 24 ou 48h d'avec le milieu bactérien ne modifie pas notablement l'effet obtenu.

L'association de l'extrait de myrte et du fluorinol permet d'obtenir un gain d'activité notable par rapport aux produits testés seuls. Ces expériences prouvent la synergie de l'association de ces deux produits dans la réduction de la formation du biofilm. Par ailleurs, le fait d'augmenter le nombre de traitements (3 traitements pour le temps 24h et 6 pour le temps 48h) induit une augmentation de l'activité de la combinaison.

## Revendications

1. Composition comprenant un extrait de myrte et au moins un sel de fluor, **caractérisé en ce que** l'extrait de myrte comprend entre 1 et 5 % de flavonoïdes, entre 0,5 et 10 % de tanins, entre 0,5 et 5 % de triterpènes en poids sec par rapport au poids total de la matière sèche dudit extrait.

2. Composition selon la revendication 1, destinée à une application buccale.

3. Composition selon la revendication 1 ou 2, dans laquelle le sel de fluor est un composé de formule (I) suivante : 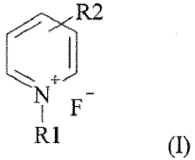 dans laquelle :
R1 représente un atome d'hydrogène ou un groupe (C₁-C₆)alkyle ;
R2 représente un groupe CH₂OH, CO₂((C₁-C₆)alkyle), CONH(CH₂)₂OH ou 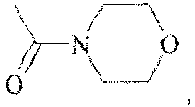 et se trouve en position 2 ou 3 par rapport à l'azote.

4. Composition selon la revendication 3, dans laquelle le sel de fluor est le fluorhydrate de nicométhanol.

5. Composition selon l'une quelconque des revendications 1 à 4, dans laquelle l'extrait de myrte est à une concentration comprise entre 0,005 % et 3 % en poids d'extrait sec de myrte par rapport au poids total de la composition.

6. Composition selon l'une quelconque des revendications 1 à 5, dans laquelle le sel de fluor est à une concentration comprise entre 1 % et 10 % en poids de sel de fluor par rapport au poids total de la composition.

7. Composition selon l'une quelconque des revendications 1 à 5, dans laquelle le sel de fluor est à une concentration comprise entre 50 ppm et 10 000 ppm de fluor.

8. Composition selon l'une quelconque des revendications 1 à 7, sous forme d'une pâte dentifrice, d'un gel, d'un spray ou d'un bain de bouche.

9. Composition selon l'une quelconque des revendications 1 à 8, pour son utilisation en tant que médicament.

10. Composition pour son utilisation selon la revendication 9, pour la prévention de la formation de la plaque dentaire et/ou la réduction de la plaque dentaire.

## Patentansprüche

1. Zusammensetzung umfassend einen Myrtenextrakt und mindestens ein Fluorsalz, **dadurch gekennzeichnet, dass** der Myrtenextrakt zwischen 1 und 5 % Flavonoide, zwischen 0,5 und 10 % Tannine, zwischen 0,5 und 5 % Triterpene in Trockenmasse bezogen auf das Gesamtgewicht der Trockenmasse des Extraktes enthält.

2. Zusammensetzung nach Anspruch 1, bestimmt für die orale Anwendung.

3. Zusammensetzung nach Anspruch 1 oder 2, wobei das Fluorsalz eine Verbindung der folgenden Formel (I) ist: 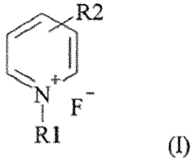 wobei:
R1 für ein Wasserstoffatom oder eine (C₁-C₆)Alkylgruppe steht;
R2 für eine CH₂OH-, CO₂((C₁-C₆)Alkyl)-, CONH(CH₂)₂OH-Gruppe
oder 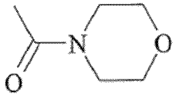
steht und sich an Position 2 oder 3 in Bezug auf den Stickstoff befindet.

4. Zusammensetzung nach Anspruch 3, wobei das Fluorsalz Nicomethanol-Hydrofluorid ist.

5. Zusammensetzung nach einem der Ansprüche 1 bis 4, wobei der Myrtenexrakt eine Konzentration zwischen 0,005 % und 3 % in Trockenmasse des Myrtenextrakts bezogen auf das Gesamtgewicht der Zusammensetzung aufweist.

6. Zusammensetzung nach einem der Ansprüche 1 bis 5, wobei das Fluorsalz eine Konzentration zwischen 1 % und 10 % in Trockenmasse des Fluorsalzes bezogen auf das Gesamtgewicht der Zusammensetzung aufweist.

7. Zusammensetzung nach einem der Ansprüche 1 bis 5, wobei das Fluorsalz eine Konzentration zwischen 50 ppm und 10.000 ppm Fluor aufweist.

8. Zusammensetzung nach einem der Ansprüche 1 bis 7 in Form einer Zahnpasta, eines Gels, eines Sprays oder einer Mundspülung.

9. Zusammensetzung nach einem der Ansprüche 1 bis 8 zur Verwendung als Medikament.

10. Zusammensetzung zur Verwendung nach Anspruch 9 zur Vorbeugung vor der Bildung von Zahnbelag und/oder zur Verringerung des Zahnbelags.

## Claims

1. A composition comprising a myrtle extract and at least one fluorine salt, **characterized in that** the myrtle extract comprises between 1 and 5% flavonoids, between 0.5 and 10% tannins, between 0.5 and 5% triterpenes by dry weight relative to the total weight of the dry matter of said extract.

2. The composition as claimed in claim 1, for oral application.

3. The composition as claimed in claim 1 or 2, wherein the fluorine salt is a compound of the following formula (I): 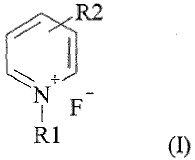 wherein:
R1 represents a hydrogen atom or 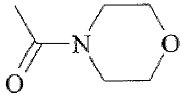 a (C1-C6)alkyl group;
R2 is CH2OH, CO2((C1-C6)alkyl), CONH(CH2)2OH or , and is in position 2 or 3 relative to the nitrogen.

4. The composition as claimed in claim 3, wherein the fluorine salt is nicomethanol hydrofluoride.

5. The composition as claimed in any one of claims 1 to 4, wherein the myrtle extract is at a concentration of between 0.005% and 3% by weight of myrtle dry extract relative to the total weight of the composition.

6. The composition as claimed in any one of claims 1 to 5, wherein the fluorine salt is at a concentration of between 1% and 10% by weight of fluorine salt relative to the total weight of the composition.

7. The composition as claimed in any one of claims 1 to 5, wherein the fluorine salt is at a concentration between 50 ppm and 10 000 ppm fluorine.

8. The composition as claimed in any one of claims 1 to 7, in the form of a toothpaste, a gel, a spray or a mouthwash.

9. The composition as claimed in any one of claims 1 to 8, for use as a medicinal product.

10. The composition for use as claimed in claim 9, for the prevention of dental plaque formation and/or reduction of dental plaque.
